(19) Europäisches Patentamt
European Patent Office
Office européen des brevets

(11) **EP 4 403 106 A1**

(12) **EUROPEAN PATENT APPLICATION**
published in accordance with Art. 153(4) EPC

(43) Date of publication:
**24.07.2024 Bulletin 2024/30**

(21) Application number: **22870283.3**

(22) Date of filing: **14.09.2022**

(51) International Patent Classification (IPC):
**A61B 5/1455** (2006.01)  **A61B 5/00** (2006.01)
**G16H 50/20** (2018.01)

(52) Cooperative Patent Classification (CPC):
**A61B 5/00; A61B 5/1455; G16H 50/20**

(86) International application number:
**PCT/KR2022/013738**

(87) International publication number:
**WO 2023/043199 (23.03.2023 Gazette 2023/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.09.2021  KR 20210124275**

(71) Applicant: **Sky Labs Inc.
Seongnam-si, Gyeonggi-do 13486 (KR)**

(72) Inventors:
• **CHANG, Hyung Min
  Yongin-si, Gyeonggi-do 16843 (KR)**

• **KIM, Hae Na
  Seongnam-si, Gyeonggi-do 13466 (KR)**
• **LEE, Min Hyung
  Bucheon-si, Gyeonggi-do 14600 (KR)**
• **KIM, Chang Hyun
  Seoul 05372 (KR)**
• **CHOI, Chang Woo
  Uiwang-si, Gyeonggi-do 16000 (KR)**
• **LEE, Byung Hwan
  Yongin-si, Gyeonggi-do 16981 (KR)**

(74) Representative: **Loyer & Abello
9, rue Anatole de la Forge
75017 Paris (FR)**

(54) **OXYGEN SATURATION ESTIMATION SYSTEM USING PPG SIGNAL SENSING RING**

(57)     Provided is a system for estimating oxygen saturation by using a photoplethysmography (PPG) signal sensing ring. The system includes a server, wherein the server includes a signal quality classification component configured to classify qualities of a first wavelength PPG signal and a second wavelength PPG signal as good or bad, and an oxygen saturation estimation component configured to estimate oxygen saturation from the first wavelength PPG signal and the second wavelength PPG signal, the PPG signal sensing ring includes a plurality of sensors at different locations, each of the plurality of sensors is configured to measure the first wavelength PPG signal and the second wavelength PPG signal, each of the plurality of sensors includes a first wavelength light source, a second wavelength light source, and a photoelectric conversion device, and the terminal includes a sensor selection component configured to select, from among the plurality of sensors, as a sensor for measuring the first wavelength PPG signal and the second wavelength PPG signal, a sensor that measured a combination of a first wavelength test PPG signal and a second wavelength test PPG signal, which has a highest signal quality from among a plurality of first wavelength test PPG signals and a plurality of second wavelength test PPG signals.

FIG. 3

## Description

Technical Field

The present disclosure relates to a system for estimating oxygen saturation. More particularly, the present disclosure relates to a system for estimating oxygen saturation by using a photoplethysmography (PPG) signal sensing ring.

Background Art

Oxygen saturation denotes a percentage of hemoglobin saturated in combination with oxygen in all the hemoglobin in the body. The oxygen saturation is generally 95 % to 100 %, and when the oxygen saturation is 90 % or less, hypoxemia may break out, and when the oxygen saturation is 80 % or less, several tissues of the body may be severely damaged. There is a need for a method of checking a health condition by easily measuring oxygen saturation in everyday life without having to visit a hospital.

Disclosure

Technical Problem

Provided is a system for estimating oxygen saturation by using a photoplethysmography (PPG) signal sensing ring.

Technical Solution

A system for estimating oxygen saturation by using a photoplethysmography (PPG) signal sensing ring, according to an embodiment of the present disclosure, includes a server, wherein the server includes a signal quality classification component configured to classify qualities of a first wavelength PPG signal and a second wavelength PPG signal as good or bad, and an oxygen saturation estimation component configured to estimate oxygen saturation from the first wavelength PPG signal and the second wavelength PPG signal, the first wavelength PPG signal and the second wavelength PPG signal are measured by using the PPG signal sensing ring, the server receives the first wavelength PPG signal and the second wavelength PPG signal from the PPG signal sensing ring through a terminal, the PPG signal sensing ring includes a plurality of sensors at different locations, each of the plurality of sensors is configured to measure the first wavelength PPG signal and the second wavelength PPG signal, each of the plurality of sensors includes a first wavelength light source, a second wavelength light source, and a photoelectric conversion device, and the terminal includes a sensor selection component configured to select, from among the plurality of sensors, as a sensor for measuring the first wavelength PPG signal and the second wavelength PPG signal, a sensor that measured a combination of a first wavelength test PPG signal and a second wavelength test PPG signal, which has a highest signal quality from among a plurality of first wavelength test PPG signals and a plurality of second wavelength test PPG signals.

According to some embodiments, signal qualities of the plurality of test PPG signals may be evaluated by at least one of a magnitude of an acceleration signal, a signal-to-noise ratio, and a ratio of a direct current (DC) component size to an alternating current (AC) component size.

According to some embodiments, the server may further include an oxygen saturation index calculation component configured to calculate an oxygen saturation index, wherein the oxygen saturation index may be defined by a ratio of a time when the qualities of the first wavelength PPG signal and the second wavelength PPG signal are classified as good by the signal quality classification component to a time when the qualities of the first wavelength PPG signal and the second wavelength PPG signal are classified as good and the oxygen saturation is outside a normal range.

According to some embodiments, the terminal may further include a light source control component configured to control the first wavelength light source and the second wavelength light source of each of the plurality of sensors such that a DC component of each of the plurality of first wavelength test PPG signals measured respectively by using the plurality of sensors is within a first predetermined range and a DC component of each of the plurality of second wavelength test PPG signals measured respectively by using the plurality of sensors is within a second predetermined range.

According to some embodiments, the controlling of the first wavelength light source and the second wavelength light source by the light source control component and the selecting of the sensor by the sensor selection component may be sequentially performed, and the controlling of the first wavelength light source and the second wavelength light source by the light source control component and the selecting of the sensor by the sensor selection component may be periodically performed.

A method of estimating oxygen saturation by using a photoplethysmography (PPG) signal sensing ring, according to an embodiment of the present disclosure, includes receiving a first wavelength PPG signal and a second wavelength PPG signal from the PPG signal sensing ring through a terminal, classifying qualities of the first wavelength PPG signal and the second wavelength PPG signal as good or bad, and estimating oxygen saturation from the first wavelength PPG signal and the second wavelength PPG signal, wherein the PPG signal sensing ring includes a plurality of sensors at different locations, each of the plurality of sensors is configured to measure the first wavelength PPG signal and the second wavelength PPG signal, each of the plurality of sensors includes a first wavelength light source, a second wavelength light source, and a photoelectric conversion device, and the terminal includes a sensor selection component configured to select, from among the plurality of sensors, as a sensor for measuring the first wavelength PPG signal and the second wavelength PPG signal, a sensor that measured a combination of a first wavelength test PPG signal and a second wavelength test PPG signal, which has a highest signal quality from among a plurality of first wavelength test PPG signals and a plurality of second wavelength test PPG signals.

According to some embodiments, signal qualities of the plurality of test PPG signals may be evaluated by at least one of a magnitude of an acceleration signal, a signal-to-noise ratio, and a ratio of a direct current (DC) component size to an alternating current (AC) component size.

According to some embodiments, the method may further include calculating an oxygen saturation index, wherein the oxygen saturation index may be defined by a ratio of a time when the qualities of the first wavelength PPG signal and the second wavelength PPG signal are classified as good by the signal quality classification component to a time when the qualities of the first wavelength PPG signal and the second wavelength PPG signal are classified as good and the oxygen saturation is outside a normal range.

According to some embodiments, the terminal may further include a light source control component configured to control the first wavelength light source and the second wavelength light source of each of the plurality of sensors such that a DC component of each of the plurality of first wavelength test PPG signals measured respectively by using the plurality of sensors is within a first predetermined range and a DC component of each of the plurality of second wavelength test PPG signals measured respectively by using the plurality of sensors is within a second predetermined range.

According to some embodiments, the controlling of the first wavelength light source and the second wavelength light source by the light source control component and the selecting of the sensor by the sensor selection component may be sequentially performed, and the controlling of the first wavelength light source and the second wavelength light source by the light source control component and the selecting of the sensor by the sensor selection component may be periodically performed.

Advantageous Effects

Provided is a system for estimating oxygen saturation by using a photoplethysmography (PPG) signal sensing ring. According to the present disclosure, oxygen saturation may be continuously and conveniently monitored in everyday life. According to the present disclosure, the PPG signal sensing ring may include a plurality of sensors at different locations. Among the plurality of sensors, a sensor in which signal qualities of a first wavelength PPG signal and a second wavelength PPG signal are excellent may be selected. Accordingly, a sensor may be selected according to different blood vessel locations and shapes of a user.

Description of Drawings

FIG. 1 is a 3-dimensional view of a photoplethysmography (PPG) signal sensing ring according to an embodiment of the present disclosure.
FIG. 2 is an exploded 3-dimensional view of a PPG signal sensing ring according to an embodiment of the present disclosure.
FIG. 3 is a block diagram of a system for estimating oxygen saturation by using a PPG signal sensing ring, according to an embodiment of the present disclosure.
FIG. 4 is a flowchart of a method of estimating oxygen saturation by using a PPG signal sensing ring.
FIG. 5 is a flowchart of a method of setting a sensor of a PPG signal sensing ring.

Mode for Invention

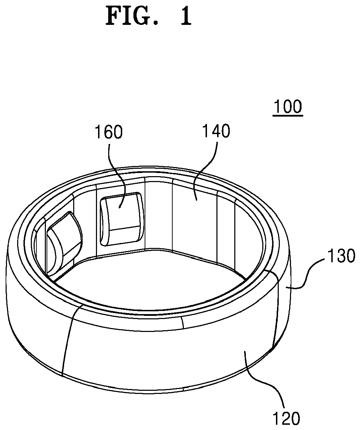

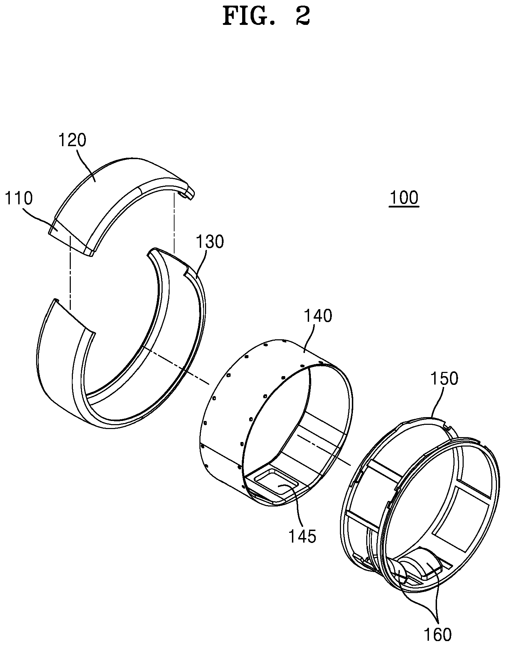

FIG. 1 is a 3-dimensional view of a photoplethysmography (PPG) signal sensing ring 100 according to an embodiment of the present disclosure. FIG. 2 is an exploded 3-dimensional view of the PPG signal sensing ring 100 according to an embodiment of the present disclosure.

Referring to FIGS. 1 and 2, the PPG signal sensing ring 100 may include an outer electrode 130, an inner electrode 140, an insulating unit 150, a top cover 110, an operation display unit 120, and a plurality of sensors 160.

The outer electrode 130 may have an arch shape. The outer electrode 130 may include a conductor and function as an electrode for measuring electrocardiogram (ECG). Also, the outer electrode 130 may form the outer appearance of the PPG signal sensing ring 100 and be in contact with the body of a user.

The inner electrode 140 may have a ring shape and include a plurality of openings 145 for the plurality of sensors 160. The inner electrode 140 may include a conductor and function as an electrode for measuring ECG. Also, the inner electrode 140 may form the inner appearance of the PPG signal sensing ring 100 and be in contact with a finger of the user.

The insulating unit 150 may be provided between the outer electrode 130 and the inner electrode 140. The insulating unit 150 may enable electric insulation between the outer electrode 130 and the inner electrode 140.

The top cover 110 may have an arc shape and form a ring shape together with the outer electrode 130. The top cover 110 may form the outer appearance of the PPG signal sensing ring 100.

The operation display unit 120 may be combined with the top cover 110. The operation display unit 120 may include a plurality of light-emitting diodes (LEDs), for example, a green LED and a red LED. The operation display unit 120 may display an operation of the PPG signal sensing ring 100 by using the plurality of LEDs. For example, the green LED may be turned on for 2 seconds to indicate the start of measurement, and the green LED may be turned on for 1 second to indicate the end of the measurement. Also, the red LED may be repeatedly turned on and off at intervals of 1 second to indicate malfunction.

The plurality of sensors 160 may be arranged to be in contact with the finger of the user. The plurality of sensors 160 may be respectively located in the plurality of openings 145 of the inner electrode 140 and protrude from a surface of the inner electrode 140. The plurality of sensors 160 may be configured to obtain a plurality of first wavelength PPG signals and a plurality of second wavelength PPG signals at different locations. Each sensor 160 may include a first wavelength light source and a second wavelength light source, which have different wavelengths, and a photoelectric conversion device.

According to some embodiments, although not shown in FIGS. 1 and 2, the PPG signal sensing ring 100 may further include an acceleration sensor arranged between the outer electrode 130 and the inner electrode 140.

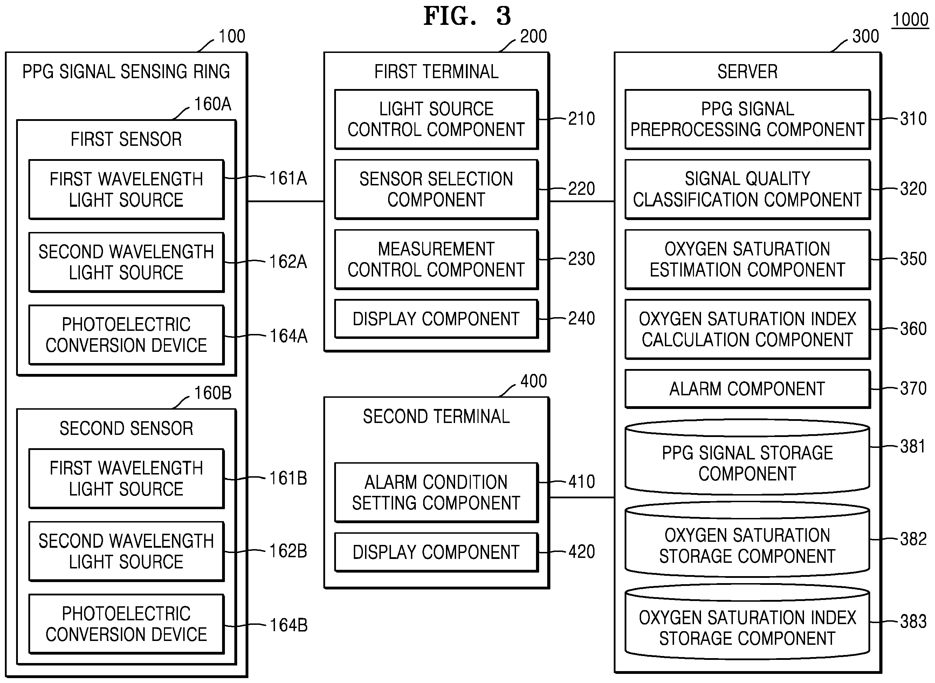

FIG. 3 is a block diagram of a system 1000 for estimating oxygen saturation by using a PPG signal sensing ring, according to an embodiment of the present disclosure.

Referring to FIG. 3, the system 1000 may include the PPG signal sensing ring 100, a first terminal 200, a server 300, and a second terminal 400.

The PPG signal sensing ring 100 may include a plurality of sensors, for example, a first sensor 160A and a second sensor 160B. In FIG. 3, the PPG signal sensing ring 100 includes two sensors, but the number of sensors included in the PPG signal sensing ring 100 is not limited to two. According to some embodiments, although not shown in FIG. 3, the PPG signal sensing ring 100 may further include an acceleration sensor.

The first sensor 160A may include a first wavelength light source 161A, a second wavelength light source 162A, and a photoelectric conversion device 164A. The first sensor 160A may detect a first wavelength PPG signal by using the first wavelength light source 161A and the photoelectric conversion device 164A. The first sensor 160A may also detect a second wavelength PPG signal by using the second wavelength light source 162A and the photoelectric conversion device 164A. The first wavelength light source 161A may include, for example, a red LED, the second wavelength light source 162A may include, for example, an infrared LED, and the photoelectric conversion device 164A may include a photodiode.

The second sensor 160B may include a first wavelength light source 161B, a second wavelength light source 162B, and a photoelectric conversion device 164B. The second sensor 160B may detect the first wavelength PPG signal by using the first wavelength light source 161B and the photoelectric conversion device 164B. The second sensor 160B may also detect the second wavelength PPG signal by using the second wavelength light source 162B and the photoelectric conversion device 164B. The first wavelength light source 161B may include, for example, a red LED, the second wavelength light source 162B may include, for example, an infrared LED, and the photoelectric conversion device 164B may include a photodiode.

The acceleration sensor may detect movement of the user by measuring acceleration of the PPG signal sensing ring 100.

The first terminal 200 may be used by the user. The first terminal 200 may include, for example, a smartphone or a tablet personal computer (PC). The first terminal 200 may be connected to the PPG signal sensing ring 100 wirelessly or via wires. For example, the first terminal 200 may be connected to the PPG signal sensing ring 100 by using Bluetooth or Wi-Fi. The first terminal 200 may be connected to the server 300 wirelessly or via wires. For example, the first terminal 200 may be connected to the server 300 via Wi-Fi or mobile telecommunication technology such as 3rd generation (3G), long-term evolution (LTE), or 5th generation (5G).

The first terminal 200 may include a light source control component 210, a sensor selection component 220, a measurement control component 230, and a display component 240. An application may be installed in the first terminal 200. The light source control component 210, the sensor selection component 220, the measurement control component 230, and the display component 240 may be realized by an application. Alternatively, the first terminal 200 may access a website, and the light source control component 210, the sensor selection component 220, the measurement control component 230, and the display component 240 may be realized by the website.

The light source control component 210 may control the first wavelength light source 161A and the second wavelength light source 162A of the first sensor 160A, and the first wavelength light source 161B and the second wavelength light source 162B of the second sensor 160B such that a direct current (DC) component of each of two first wavelength test PPG signals received from the first sensor 160A and the second sensor 160B is within a first predetermined range and a DC component of each of two second wavelength test PPG signals received from the first sensor 160A and the second sensor 160B is within a second predetermined range.

After the light source control component 210 controls the first wavelength light source 161A and the second wavelength light source 162A of the first sensor 160A, and the first wavelength light source 161B and the second wavelength light source 162B of the second sensor 160B, the sensor selection component 220 may select one of the first sensor 160A and the second sensor 160B as a sensor for measuring a first wavelength PPG signal and a second wavelength PPG signal afterwards. The sensor selection component 220 may select, from among the first sensor 160A and the second sensor 160B, a sensor that measured a combination of a first wavelength test PPG signal and a second wavelength test PPG signal, which has the highest signal quality, from among two first wavelength test PPG signals and two second wavelength test PPG signals from the first sensor 160A and the second sensor 160B. For example, a sensor that measured a combination of a first wavelength test PPG signal and a second wavelength test PPG signal, which has the highest average of signal quality, from among the two first wavelength test PPG signals and the two second wavelength test PPG signals, may be selected. Signal quality may be evaluated by at least one of the magnitude of an acceleration signal, a signal-to-noise ratio (SNR), and a ratio of a DC component size to an alternating current (AC) component size. The signal quality may be high when the magnitude of the acceleration signal is low, the SNR is high, and the ratio of a DC component size to an AC component size is high. Then, the sensor selected by the sensor selection component 220 may measure the first wavelength PPG signal and the second wavelength PPG signal.

The controlling of the first wavelength light source 161A and the second wavelength light source 162A of the first sensor 160A, and the first wavelength light source 161B and the second wavelength light source 162B of the second sensor 160B by the light source control component 210, and the selecting of a sensor by the sensor selection component 220 may be sequentially performed. In other words, the sensor selection component 220 may select a sensor after the light source control component 210 controls the first wavelength light source 161A and the second wavelength light source 162A of the first sensor 160A, and the first wavelength light source 161B and the second wavelength light source 162B of the second sensor 160B.

The controlling of the first wavelength light source 161A and the second wavelength light source 162A of the first sensor 160A, and the first wavelength light source 161B and the second wavelength light source 162B of the second sensor 160B by the light source control component 210, and the selecting of a sensor by the sensor selection component 220 may be periodically performed. The PPG signal sensing ring 100 may move or rotate with respect to the finger, and thus, the light source control component 210 may periodically control the first wavelength light source 161A and the second wavelength light source 162A of the first sensor 160A, and the first wavelength light source 161B and the second wavelength light source 162B of the second sensor 160B, and the sensor selection component 220 may periodically select a sensor.

The measurement control component 230 may select a measurement mode or start or end measurement, according to the user's input. For example, the user may select the measurement mode to be one of a self-check mode and a background mode by using the measurement control component 230. In the self-check mode, the user may start or end the measurement by using the measurement control component 230. In the background mode, the measurement starts and continues regardless of the user's input. In the background mode, the user may set or change a measurement cycle. According to some embodiments, the measurement control component 230 may be included in the PPG signal sensing ring 100. In other words, the user may select the measurement mode, or may start or end the measurement by using the measurement control component 230. According to some embodiments, the measurement control component 230 may be included in the server 300. In other words, a service provider may select the measurement mode, or may start or end the measurement by using the measurement control component 230 of the server 300.

The display component 240 may display at least one of the first wavelength PPG signal and the second wavelength PPG signal measured by the PPG signal sensing ring 100 and stored in a PPG signal storage component 381 of the server 300, a measurement time of the first wavelength PPG signal and the second wavelength PPG signal stored in the PPG signal storage component 381 of the server 300, a signal quality classification result obtained by a signal quality classification component 320 of the server 300 and stored in an oxygen saturation storage component 382 of the server 300, oxygen saturation estimated by an oxygen saturation estimation component 350 of the server 300 and stored in the oxygen saturation storage component 382 of the server 300, and an oxygen saturation index calculated by an oxygen saturation index calculation component 360 of the server 300 and stored in an oxygen saturation index storage component 383.

The server 300 may include a PPG signal preprocessing component 310, the signal quality classification component 320, the oxygen saturation estimation component 350, the PPG signal storage component 381, and the oxygen saturation storage component 382. According to some embodiments, the server 300 may further include the oxygen saturation index calculation component 360 and the oxygen saturation index storage component 383. According to some embodiments, the server 300 may further include an alarm component 370.

The PPG signal preprocessing component 310 may preprocess the first wavelength PPG signal and the second wavelength PPG signal received by the server 300 from the selected sensor of the PPG signal sensing ring 100 through the first terminal 200. For example, a low-pass filter, a high-pass filter, and normalization may be used to preprocess the first wavelength PPG signal and the second wavelength PPG signal. According to some embodiments, the PPG signal preprocessing component 310 may preprocess the acceleration signal received by the server 300 from the acceleration sensor of the PPG signal sensing ring 100 through the first terminal 200.

The signal quality classification component 320 may classify the signal qualities of the first wavelength PPG signal and the second wavelength PPG signal as good or bad. According to some embodiments, the signal quality classification component 320 may refer to the acceleration signal to classify the signal qualities of the first wavelength PPG signal and the second wavelength PPG signal as one of good and bad.

It may be determined that oxygen saturation estimated from the first wavelength PPG signal and the second wavelength PPG signal, classified as being bad quality, is not reliable, and the oxygen saturation may not be displayed by the display component 240 of the first terminal 200. It may be determined that oxygen saturation estimated from the first wavelength PPG signal and the second wavelength PPG signal, classified being good quality, is reliable, and the oxygen saturation may be displayed by the display component 240 of the first terminal 200. According to an alternative embodiment, the oxygen saturation estimated from the first wavelength PPG signal and the second wavelength PPG signal may be displayed by the display component 240 of the first terminal 200, together with the signal quality classification result, regardless of the signal quality classification result. Also, the oxygen saturation estimated from the first wavelength PPG signal and the second wavelength PPG signal may be displayed by a display component 420 of the second terminal 400, together with the signal quality classification result, regardless of the signal quality classification result.

According to some embodiments, the signal quality classification component 320 may classify quality of a PPG signal as good or bad by using a deep learning model. The deep learning model used by the signal quality classification component 320 may include a convolution neural network (CNN), a long short-term memory (LSTM), a fully-connected network (FCN), an encoder, a decoder, or a combination thereof. A method of classifying signal quality is not limited to the method described in the present specification.

The oxygen saturation estimation component 350 may estimate the oxygen saturation from the first wavelength PPG signal and the second wavelength PPG signal. Various methods may be used to estimate the oxygen saturation.

For example, a value of R may be first calculated according to Equation 1 below.

[Equation 1]

$$R = ((AC_R)/(DC_R))/((AC_{IR})/(DC_{IR}))$$

Here, $AC_R$ denotes the size of an AC component of the first wavelength PPG signal, $DC_R$ denotes the size of a DC component of the first wavelength PPG signal, $AC_{IR}$ denotes the size of an AC component of the second wavelength PPG signal, and $DC_{IR}$ denotes the size of a DC component of the second wavelength PPG signal.

Then, oxygen saturation may be calculated from the value of R, according to Equation 2 below.

[Equation 2]

Oxygen Saturation $= C_0 - C_1 R$

$C_0$ and $C_1$ are constants that may be determined by using linear regression.

In Equation 2, the oxygen saturation is represented as a primary expression for the value of R, but the oxygen saturation may be represented as a polynomial expression of a degree greater than 1 for the value of R, for example, a quadratic or cubic expression. A method of estimating oxygen saturation is not limited to the method described in the present specification.

The oxygen saturation index calculation component 360 may calculate the oxygen saturation index. The oxygen saturation index may be defined based on a time when the qualities of the first wavelength PPG signal and the second wavelength PPG signal are classified as good by the signal quality classification component 320, and a time when the qualities of the first wavelength PPG signal and the second wavelength PPG signal are classified as good by the signal quality classification component 320 and the oxygen saturation estimated by the oxygen saturation estimation component 350 is outside a normal range. For example, the oxygen saturation index may be defined as a ratio of the time when the qualities of the first wavelength PPG signal and the second wavelength PPG signal are classified as good by the signal quality classification component 320 to the time when the qualities of the first wavelength PPG signal and the second wavelength PPG signal are classified as good by the signal quality classification component 320 and the oxygen saturation estimated by the oxygen saturation estimation component 350 is outside the normal range. In other words, the oxygen saturation index may denote a ratio of a time when oxygen saturation is outside the normal range to a time when reliable oxygen saturation is obtainable.

The alarm component 370 may transmit an alarm to at least one of the first terminal 200 and the second terminal 400 when at least one of the oxygen saturation estimated by the oxygen saturation estimation component 350 and the oxygen saturation index calculated by the oxygen saturation index calculation component 360 satisfies an alarm condition set by an alarm condition setting component 410 of the second terminal 400.

The PPG signal storage component 381 may store the first wavelength PPG signal and the second wavelength PPG signal received by the server 300 from the selected sensor of the PPG signal sensing ring 100 through the first terminal 200. The PPG signal storage component 381 may further store the measurement time of the first wavelength PPG signal and the second wavelength PPG signal. The PPG signal storage component 381 may further store the first wavelength PPG signal and the second wavelength PPG signal preprocessed by the PPG signal preprocessing component 310. The PPG signal storage component 381 may further store the acceleration signal received by the server 300 from the acceleration sensor of the PPG signal sensing ring 100 through the first terminal 200. The oxygen saturation storage component 382 may store the oxygen saturation estimated by the oxygen saturation estimation component 350. The oxygen saturation storage component 382 may further store the signal quality classification result obtained by the signal quality classification component 320. The oxygen saturation index storage component 383 may store the oxygen saturation index calculated by the oxygen saturation index calculation component 360.

The second terminal 400 may be connected to the server 300 wirelessly or via wires. For example, the second terminal 400 may be connected to the server 300 via Wi-Fi or mobile telecommunication technology such as 3G, LTE, or 5G. The second terminal 400 may be used by a doctor. The second terminal 400 may include, for example, a smartphone, a tablet PC, a computer, or a laptop computer.

The second terminal 400 may include the alarm condition setting component 410 and the display component 420. The second terminal 400 may access a website or an application may be installed in the second terminal 400. The alarm condition setting component 410 and the display component 420 may be realized by using the website or the application.

The doctor may set the alarm condition by using the alarm condition setting component 410. For example, an alarm may be set to go off when less than 90 % of oxygen saturation continues for 10 minutes or more.

The display component 420 may display at least one of the first wavelength PPG signal and the second wavelength PPG signal from the PPG signal storage component 381, the measurement time of the first wavelength PPG signal and the second wavelength PPG signal from the PPG signal storage component 381, the preprocessed first wavelength PPG signal and the preprocessed second wavelength PPG signal stored in the PPG signal storage component 381, the signal quality classification result from the oxygen saturation storage component 382, the oxygen saturation from the oxygen saturation storage component 382, and the oxygen saturation index from the oxygen saturation index storage component 383.

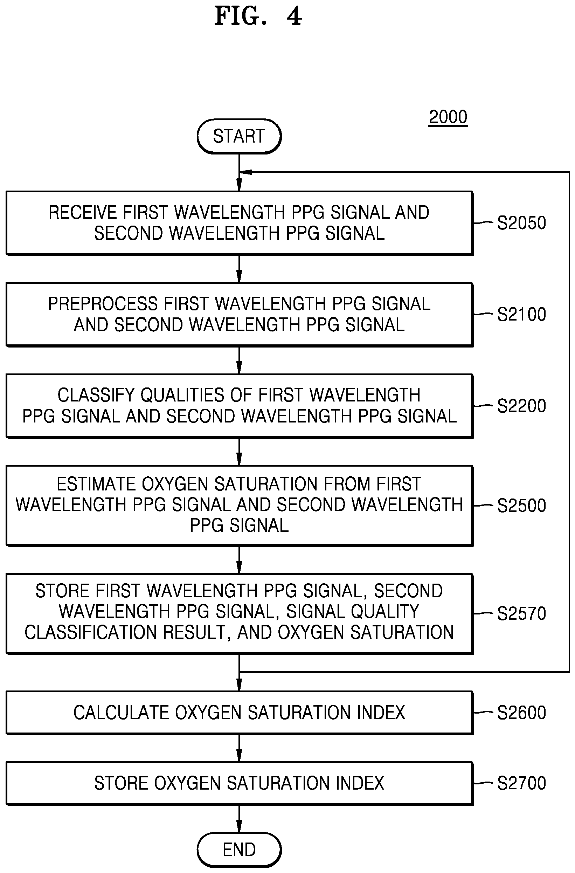

FIG. 4 is a flowchart of a method 2000 of estimating oxygen saturation by using a PPG signal sensing ring.

Referring to FIGS. 3 and 4, the server 300 may receive the first wavelength PPG signal and the second wavelength PPG signal from the selected sensor of the PPG signal sensing ring 100 through the first terminal 200 (operation S2050). Next, the PPG signal preprocessing component 310 may preprocess the first wavelength PPG signal and the second wavelength PPG signal (operation S2100). For example, a low-pass filter, a high-pass filter, and normalization may be used to preprocess the first wavelength PPG signal and the second wavelength PPG signal. According to some embodiments, the PPG signal preprocessing component 310 may further preprocess the acceleration signal received by the server 300 from the acceleration sensor of the PPG signal sensing ring 100 through the first terminal 200.

Next, the signal quality classification component 320 may classify the signal qualities of the first wavelength PPG signal and the second wavelength PPG signal as good or bad (operation S2200). The signal quality classification component 320 may refer to the acceleration signal to classify the signal qualities of the first wavelength PPG signal and the second wavelength PPG signal as good or bad. It may be determined that oxygen saturation estimated from the first wavelength PPG signal and the second wavelength PPG signal, classified as being bad quality, is not reliable, and the oxygen saturation may not be displayed by the display component 240 of the first terminal 200. It may be determined that oxygen saturation estimated from the first wavelength PPG signal and the second wavelength PPG signal, classified being good quality, is reliable, and the oxygen saturation may be displayed by the display component 240 of the first terminal 200. According to an alternative embodiment, the oxygen saturation estimated from the first wavelength PPG signal and the second wavelength PPG signal may be displayed by the display component 240 of the first terminal 200, together with the signal quality classification result, regardless of the signal quality classification result. Also, the oxygen saturation estimated from the first wavelength PPG signal and the second wavelength PPG signal may be displayed by the display component 420 of the second terminal 400, together with the signal quality classification result, regardless of the signal quality classification result.

According to some embodiments, a deep learning model may be used for the signal quality classification component 320. The deep learning model used by the signal quality classification component 320 may include CNN, LSTM, FCN, an encoder, a decoder, or a combination thereof. A method of classifying signal quality is not limited to the method described in the present specification.

Then, the oxygen saturation estimation component 350 may estimate the oxygen saturation from the first wavelength PPG signal and the second wavelength PPG signal (operation S2500). A method of estimating oxygen saturation has been described above with reference to FIG. 3.

Next, the first wavelength PPG signal, the second wavelength PPG signal, the signal quality classification result, and the oxygen saturation may be stored (operation S2570). The first wavelength PPG signal and the second wavelength PPG signal may be stored in the PPG signal storage component 381. According to some embodiments, the preprocessed first wavelength PPG signal and the preprocessed second wavelength PPG signal may be further stored in the PPG signal storage component 381. According to some embodiments, the acceleration signal may be further stored in the PPG signal storage component 381. According to some embodiments, the preprocessed acceleration signal may be further stored in the PPG signal storage component 381. The signal quality classification result and the oxygen saturation may be stored in the oxygen saturation storage component 382.

Then, the receiving of the first wavelength PPG signal and the second wavelength PPG signal (operation S2050), the preprocessing of the first wavelength PPG signal and the second wavelength PPG signal (operation S2100), the classifying of the qualities of the first wavelength PPG signal and the second wavelength PPG signal (operation S2220), the estimating of the oxygen saturation (operation S2500), and the storing (operation S2570) may be repeated.

Next, the oxygen saturation index calculation component 360 may calculate the oxygen saturation index (operation S2600). The oxygen saturation index may be defined based on the time when the qualities of the first wavelength PPG signal and the second wavelength PPG signal are classified as good by the signal quality classification component 320, and the time when the qualities of the first wavelength PPG signal and the second wavelength PPG signal are classified as good by the signal quality classification component 320 and the oxygen saturation estimated by the oxygen saturation estimation component 350 is outside the normal range. For example, the oxygen saturation index may be defined as the ratio of the time when the qualities of the first wavelength PPG signal and the second wavelength PPG signal are classified as good by the signal quality classification component 320 to the time when the qualities of the first wavelength PPG signal and the second wavelength PPG signal are classified as good by the signal quality classification component 320 and the oxygen saturation estimated by the oxygen saturation estimation component 350 is outside the normal range. In other words, the oxygen saturation index may denote the ratio of the time when oxygen saturation is outside the normal range to the time when reliable oxygen saturation is obtainable.

Next, the oxygen saturation index may be stored in the oxygen saturation index storage component 383 (operation S2700).

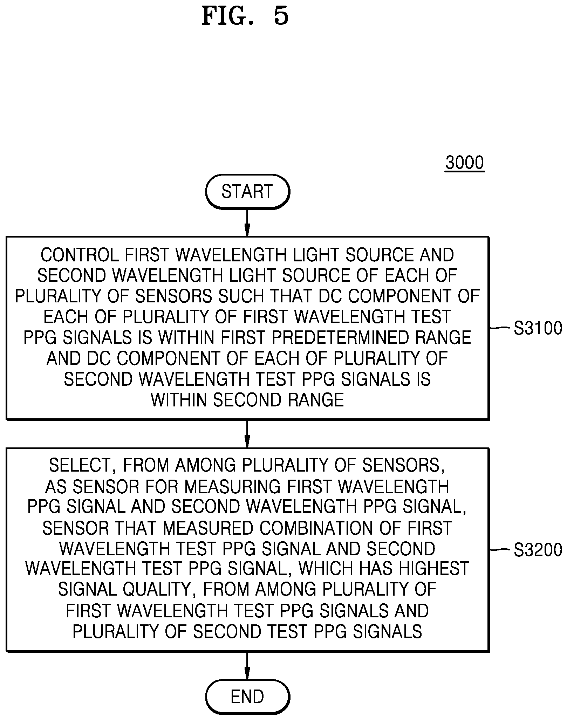

FIG. 5 is a flowchart of a method 3000 of setting a sensor of a PPG signal sensing ring.

Referring to FIGS. 3 and 5, the light source control component 210 may control the first wavelength light source 161A and the second wavelength light source 162A of the first sensor 160A, and the first wavelength light source 161B and the second wavelength light source 162B of the second sensor 160B such that the DC component of each of the two first wavelength test PPG signals received from the first sensor 160A and the second sensor 160B is within the first predetermined range and the DC component of each of the two second wavelength test PPG signals received from the first sensor 160A and the second sensor 160B is within the second predetermined range (operation S3100).

After the light source control component 210 controls the first wavelength light source 161A and the second wavelength light source 162A of the first sensor 160A, and the first wavelength light source 161B and the second wavelength light source 162B of the second sensor 160B, the sensor selection component 220 may select one of the first sensor 160A and the second sensor 160B as a sensor for measuring a first wavelength PPG signal and a second wavelength PPG signal afterwards (operation S3200). The sensor selection component 220 may select, from among the first sensor 160A and the second sensor 160B, a sensor that measured a combination of a first wavelength test PPG signal and a second wavelength test PPG signal, which has the highest signal quality, from among the two first wavelength test PPG signals and the two second wavelength test PPG signals from the first sensor 160A and the second sensor 160B. For example, a sensor that measured a combination of a first wavelength test PPG signal and a second wavelength test PPG signal, which has the highest average of signal quality, from among the two first wavelength test PPG signals and the two second wavelength test PPG signals, may be selected. Signal quality may be evaluated by at least one of the magnitude of an acceleration signal, an SNR, and a ratio of a DC component size to an AC component size. The signal quality may be high when the magnitude of the acceleration signal is low, the SNR is high, and the ratio of a DC component size to an AC component size is high. Then, the first wavelength PPG signal and the second wavelength PPG signal measured by the sensor selected by the sensor selection component 220 may be transmitted to the server 300 through the first terminal 200.

The controlling of the first wavelength light source 161A and the second wavelength light source 162A of the first sensor 160A, and the first wavelength light source 161B and the second wavelength light source 162B of the second sensor 160B by the light source control component 210 (operation S3100), and the selecting of a sensor by the sensor selection component 220 (operation S3200) may be sequentially performed. In other words, the sensor selection component 220 may select a sensor after the light source control component 210 controls the first wavelength light source 161A and the second wavelength light source 162A of the first sensor 160A, and the first wavelength light source 161 B and the second wavelength light source 162B of the second sensor 160B.

The controlling of the first wavelength light source 161A and the second wavelength light source 162A of the first sensor 160A, and the first wavelength light source 161B and the second wavelength light source 162B of the second sensor 160B by the light source control component 210 (operation S3100), and the selecting of a sensor by the sensor selection component 220 (operation S3200) may be periodically performed. The PPG signal sensing ring 100 may move or rotate with respect to the finger, and thus, the light source control component 210 may periodically control the first wavelength light source 161A and the second wavelength light source 162A of the first sensor 160A, and the first wavelength light source 161B and the second wavelength light source 162B of the second sensor 160B, and the sensor selection component 220 may periodically select a sensor.

The embodiments in the present disclosure are not intended to limit the technical ideas of the present disclosure but to describe the present disclosure, and the scope of the technical ideas of the present disclosure is not limited by these embodiments. The scope of protection of the present disclosure should be interpreted by the following claims, and all technical ideas within the scope equivalent thereto should be construed as being included in the scope of the present disclosure.

**Claims**

1. A system for estimating oxygen saturation by using a photoplethysmography (PPG) signal sensing ring, the system comprising a server, wherein the server comprises:

    a signal quality classification component configured to classify qualities of a first wavelength PPG signal and a second wavelength PPG signal as good or bad; and
    an oxygen saturation estimation component configured to estimate oxygen saturation from the first wavelength PPG signal and the second wavelength PPG signal,
    the first wavelength PPG signal and the second wavelength PPG signal are measured by using the PPG signal sensing ring, the server receives the first wavelength PPG signal and the second wavelength PPG signal from the PPG signal sensing ring through a terminal,
    the PPG signal sensing ring comprises a plurality of sensors at different locations,
    each of the plurality of sensors is configured to measure the first wavelength PPG signal and the second wavelength PPG signal,

    each of the plurality of sensors comprises a first wavelength light source, a second wavelength light source, and a photoelectric conversion device, and
    the terminal comprises a sensor selection component configured to select, from among the plurality of sensors, as a sensor for measuring the first wavelength PPG signal and the second wavelength PPG signal, a sensor that measured a combination of a first wavelength test PPG signal and a second wavelength test PPG signal, which has a highest signal quality from among a plurality of first wavelength test PPG signals and a plurality of second wavelength test PPG signals.

2. The system of claim 1, wherein signal qualities of the plurality of first wavelength test PPG signals and the plurality of second wavelength test PPG signals are evaluated by at least one of a magnitude of an acceleration signal, a signal-to-noise ratio, and a ratio of a direct current (DC) component size to an alternating current (AC) component size.

3. The system of claim 1, wherein the server further comprises an oxygen saturation index calculation component configured to calculate an oxygen saturation index,
   wherein the oxygen saturation index is defined by a ratio of a time when the qualities of the first wavelength PPG signal and the second wavelength PPG signal are classified as good by the signal quality classification component to a time when the qualities of the first wavelength PPG signal and the second wavelength PPG signal are classified as good and the oxygen saturation is outside a normal range.

4. The system of claim 1, wherein the terminal further comprises a light source control component configured to control the first wavelength light source and the second wavelength light source of each of the plurality of sensors such that a DC component of each of the plurality of first wavelength test PPG signals measured respectively by using the plurality of sensors is within a first predetermined range and a DC component of each of the plurality of second wavelength test PPG signals measured respectively by using the plurality of sensors is within a second predetermined range.

5. The system of claim 4, wherein the controlling of the first wavelength light source and the second wavelength light source by the light source control component and the selecting of the sensor by the sensor selection component are sequentially performed, and
   the controlling of the first wavelength light source and the second wavelength light source by the light source control component and the selecting of the sensor by the sensor selection component are periodically performed.

6. A method of estimating oxygen saturation by using a photoplethysmography (PPG) signal sensing ring, the method comprising:

receiving a first wavelength PPG signal and a second wavelength PPG signal from the PPG signal sensing ring through a terminal;
classifying qualities of the first wavelength PPG signal and the second wavelength PPG signal as good or bad; and
estimating oxygen saturation from the first wavelength PPG signal and the second wavelength PPG signal,
wherein the PPG signal sensing ring comprises a plurality of sensors at different locations,
each of the plurality of sensors is configured to measure the first wavelength PPG signal and the second wavelength PPG signal,
each of the plurality of sensors comprises a first wavelength light source, a second wavelength light source, and a photoelectric conversion device, and
the terminal comprises a sensor selection component configured to select, from among the plurality of sensors, as a sensor for measuring the first wavelength PPG signal and the second wavelength PPG signal, a sensor that measured a combination of a first wavelength test PPG signal and a second wavelength test PPG signal, which has a highest signal quality from among a plurality of first wavelength test PPG signals and a plurality of second wavelength test PPG signals.

7. The method of claim 6, wherein signal qualities of the plurality of first wavelength test PPG signals and the plurality of second wavelength test PPG signals are evaluated by at least one of a magnitude of an acceleration signal, a signal-to-noise ratio, and a ratio of a direct current (DC) component size to an alternating current (AC) component size.

8. The method of claim 6, further comprising calculating an oxygen saturation index,
wherein the oxygen saturation index is defined by a ratio of a time when the qualities of the first wavelength PPG signal and the second wavelength PPG signal are classified as good by the signal quality classification component to a time when the qualities of the first wavelength PPG signal and the second wavelength PPG signal are classified as good and the oxygen saturation is outside a normal range.

9. The method of claim 6, wherein the terminal further comprises a light source control component configured to control the first wavelength light source and the second wavelength light source of each of the plurality of sensors such that a DC component of each of the plurality of first wavelength test PPG signals measured respectively by using the plurality of sensors is within a first predetermined range and a DC component of each of the plurality of second wavelength test PPG signals measured respectively by using the plurality of sensors is within a second predetermined range.

10. The method of claim 9, wherein the controlling of the first wavelength light source and the second wavelength light source by the light source control component and the selecting of the sensor by the sensor selection component are sequentially performed, and
the controlling of the first wavelength light source and the second wavelength light source by the light source control component and the selecting of the sensor by the sensor selection component are periodically performed.

# FIG. 1

# FIG. 2

# FIG. 3

**PPG SIGNAL SENSING RING** — 100

**FIRST SENSOR** — 160A
- FIRST WAVELENGTH LIGHT SOURCE — 161A
- SECOND WAVELENGTH LIGHT SOURCE — 162A
- PHOTOELECTRIC CONVERSION DEVICE — 164A

**SECOND SENSOR** — 160B
- FIRST WAVELENGTH LIGHT SOURCE — 161B
- SECOND WAVELENGTH LIGHT SOURCE — 162B
- PHOTOELECTRIC CONVERSION DEVICE — 164B

**FIRST TERMINAL** — 200
- LIGHT SOURCE CONTROL COMPONENT — 210
- SENSOR SELECTION COMPONENT — 220
- MEASUREMENT CONTROL COMPONENT — 230
- DISPLAY COMPONENT — 240

**SECOND TERMINAL** — 400
- ALARM CONDITION SETTING COMPONENT — 410
- DISPLAY COMPONENT — 420

**SERVER** — 300
- PPG SIGNAL PREPROCESSING COMPONENT — 310
- SIGNAL QUALITY CLASSIFICATION COMPONENT — 320
- OXYGEN SATURATION ESTIMATION COMPONENT — 350
- OXYGEN SATURATION INDEX CALCULATION COMPONENT — 360
- ALARM COMPONENT — 370
- PPG SIGNAL STORAGE COMPONENT — 381
- OXYGEN SATURATION STORAGE COMPONENT — 382
- OXYGEN SATURATION INDEX STORAGE COMPONENT — 383

1000

EP 4 403 106 A1

# FIG. 4

2000

```
START
```

RECEIVE FIRST WAVELENGTH PPG SIGNAL AND
SECOND WAVELENGTH PPG SIGNAL — S2050

PREPROCESS FIRST WAVELENGTH PPG SIGNAL
AND SECOND WAVELENGTH PPG SIGNAL — S2100

CLASSIFY QUALITIES OF FIRST WAVELENGTH
PPG SIGNAL AND SECOND WAVELENGTH PPG SIGNAL — S2200

ESTIMATE OXYGEN SATURATION FROM FIRST
WAVELENGTH PPG SIGNAL AND SECOND WAVELENGTH — S2500
PPG SIGNAL

STORE FIRST WAVELENGTH PPG SIGNAL, SECOND
WAVELENGTH PPG SIGNAL, SIGNAL QUALITY — S2570
CLASSIFICATION RESULT, AND OXYGEN SATURATION

CALCULATE OXYGEN SATURATION INDEX — S2600

STORE OXYGEN SATURATION INDEX — S2700

```
END
```

# FIG. 5

3000

START

CONTROL FIRST WAVELENGTH LIGHT SOURCE AND
SECOND WAVELENGTH LIGHT SOURCE OF EACH OF
PLURALITY OF SENSORS SUCH THAT DC COMPONENT OF
EACH OF PLURALITY OF FIRST WAVELENGTH TEST
PPG SIGNALS IS WITHIN FIRST PREDETERMINED RANGE
AND DC COMPONENT OF EACH OF PLURALITY OF
SECOND WAVELENGTH TEST PPG SIGNALS IS
WITHIN SECOND RANGE ——S3100

SELECT, FROM AMONG PLURALITY OF SENSORS,
AS SENSOR FOR MEASURING FIRST WAVELENGTH
PPG SIGNAL AND SECOND WAVELENGTH PPG SIGNAL,
SENSOR THAT MEASURED COMBINATION OF FIRST
WAVELENGTH TEST PPG SIGNAL AND SECOND
WAVELENGTH TEST PPG SIGNAL, WHICH HAS HIGHEST
SIGNAL QUALITY, FROM AMONG PLURALITY OF
FIRST WAVELENGTH TEST PPG SIGNALS AND
PLURALITY OF SECOND TEST PPG SIGNALS ——S3200

END

## INTERNATIONAL SEARCH REPORT

International application No.

**PCT/KR2022/013738**

### A. CLASSIFICATION OF SUBJECT MATTER

**A61B 5/1455**(2006.01)i; **A61B 5/00**(2006.01)i; **G16H 50/20**(2018.01)i

According to International Patent Classification (IPC) or to both national classification and IPC

### B. FIELDS SEARCHED

Minimum documentation searched (classification system followed by classification symbols)

A61B 5/1455(2006.01); A61B 5/00(2006.01); A61B 5/024(2006.01); A61B 5/0245(2006.01); A61B 5/145(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 광전용적맥파 (Photoplethysmography), 반지 (ring), 산소포화도 (oxygen saturation), 분류 (classification), 품질 (quality), 선택 (selection)

### C. DOCUMENTS CONSIDERED TO BE RELEVANT

| Category* | Citation of document, with indication, where appropriate, of the relevant passages | Relevant to claim No. |
|---|---|---|
| Y | KR 10-2020-0091625 A (SAMSUNG ELECTRONICS CO., LTD.) 31 July 2020 (2020-07-31) See paragraphs [0031]-[0075]; claims 1, 2 and 6; and figure 1. | 1-10 |
| Y | KR 10-2017-0004607 A (SAMSUNG ELECTRONICS CO., LTD.) 11 January 2017 (2017-01-11) See claim 10. | 1-10 |
| Y | JP 2007-020836 A (KONICA MINOLTA SENSING INC.) 01 February 2007 (2007-02-01) See paragraphs [0033] and [0041]. | 3,8 |
| Y | KR 10-2015-0082038 A (SAMSUNG ELECTRONICS CO., LTD.) 15 July 2015 (2015-07-15) See paragraph [0074]; and claims 5-11 and 14. | 4,5,9,10 |
| A | JP 2007-054497 A (SHARP CORP.) 08 March 2007 (2007-03-08) See entire document. | 1-10 |

☐ Further documents are listed in the continuation of Box C.  ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **29 December 2022** | **29 December 2022** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

<div align="center">

**INTERNATIONAL SEARCH REPORT**
**Information on patent family members**

</div>

International application No.

**PCT/KR2022/013738**

| Patent document cited in search report | | | Publication date (day/month/year) | Patent family member(s) | | | Publication date (day/month/year) |
|---|---|---|---|---|---|---|---|
| KR | 10-2020-0091625 | A | 31 July 2020 | US | 11419529 | B2 | 23 August 2022 |
| | | | | US | 2020-0229743 | A1 | 23 July 2020 |
| KR | 10-2017-0004607 | A | 11 January 2017 | CN | 106308776 | A | 11 January 2017 |
| | | | | EP | 3111834 | A1 | 04 January 2017 |
| | | | | EP | 3111834 | B1 | 07 July 2021 |
| | | | | KR | 10-2434698 | B1 | 22 August 2022 |
| | | | | US | 10420470 | B2 | 24 September 2019 |
| | | | | US | 11229363 | B2 | 25 January 2022 |
| | | | | US | 2017-0000350 | A1 | 05 January 2017 |
| | | | | US | 2019-0365231 | A1 | 05 December 2019 |
| JP | 2007-020836 | A | 01 February 2007 | JP | 4710084 | B2 | 29 June 2011 |
| KR | 10-2015-0082038 | A | 15 July 2015 | US | 2015-0190077 | A1 | 09 July 2015 |
| JP | 2007-054497 | A | 08 March 2007 | CN | 1919137 | A | 28 February 2007 |
| | | | | CN | 1919137 | C | 08 October 2008 |
| | | | | JP | 4607709 | B2 | 05 January 2011 |
| | | | | KR | 10-0830933 | B1 | 22 May 2008 |
| | | | | KR | 10-2007-0024432 | A | 02 March 2007 |
| | | | | US | 2007-0060807 | A1 | 15 March 2007 |

Form PCT/ISA/210 (patent family annex) (July 2022)